# EUROPÄISCHE PATENTSCHRIFT

(11) **EP 3 146 454 B1**
(45) Veröffentlichungstag und Bekanntmachung des Hinweises auf die Patenterteilung: **27.07.2022**
(21) Anmeldenummer: 15735881.3
(22) Anmeldetag: 26.06.2015
(51) Int. Cl.: A61G 13/00, G06F 3/01, A61B 6/00, A61G 7/018, A61G 12/00, A61G 13/02, A61G 17/02, G16H 40/63, A61B 6/04, H05B 47/115, G08C 17/02

(54) **STEUERUNGSVORRICHTUNG FÜR EIN MEDIZINGERÄT**
CONTROL DEVICE FOR A MEDICAL APPLIANCE
SYSTÈME DE COMMANDE POUR APPAREIL MÉDICAL

(30) Priorität: 30.06.2014 DE 102014212660
(43) Veröffentlichungstag der Anmeldung: 29.03.2017
(73) Patentinhaber: TRUMPF Medizin Systeme GmbH + Co. KG, 07318 Saalfeld (DE)
(72) Erfinder: MARKA, Rudolf, 85737 Ismaning (DE); GÜVENC, Deniz, 81737 München (DE); SCHRÖDER, Stephan, 30167 Hannover (DE); ÖZHAN, Serhan, 81379 München (DE); PÖSCH, Andreas, 30625 Hannover (DE); LOFTFIELD, Nina, 30167 Hannover (DE)
(74) Vertreter: Loustalan, Paul William
(86) Internationale Anmeldenummer: PCT/EP2015/064549
(87) Internationale Veröffentlichungsnummer: WO 2016/001089

(56) Entgegenhaltungen:
- WO-A1-2011/085813
- WO-A1-2012/041371
- US-A1- 2011 037 840
- US-A1- 2014 177 909

## Beschreibung

Die Erfindung betrifft eine Steuerungsvorrichtung für ein Medizingerät, insbesondere eine Steuerungsvorrichtung, mit der eine kontaktlose Bedienung des Medizingeräts möglich ist.

Es sind Medizingeräte, beispielsweise Operationstische oder Operationsleuchten, bekannt, deren Bedienung über Drucktasten an den Geräten selbst, an Bedientafeln an der Wand oder an Fernbedienungen erfolgt. Dabei ist es erforderlich, dass sich der Bediener in der Nähe dieser Bedienungselemente befindet. Ferner kann ein Operateur selbst die Geräte nicht bedienen, da sich die Bedienelemente üblicherweise außerhalb des sterilen Bereichs befinden und der Operateur durch ein Berühren der Bedienelemente unsteril wird.

Es ist also ein erhöhter Abstimmungsaufwand erforderlich, um die Geräte zu bedienen. Der Bediener muss erst vom Operateur angesprochen werden, er muss sich zu den Bedienelementen bewegen oder die Fernbedienung finden, ggf. die Orientierung der Bedienelemente zu dem Gerät prüfen, die Aktion starten und stoppen und ggf. nachkorrigieren.

Dokument WO 2012/041371 A1 offenbart eine Vorrichtung und ein Verfahren zum Ansteuern von Apparaten. Dabei wird eine Position, eine Bewegung oder eine Ausrichtung eines Teils eines menschlichen Körpers oder eines Objekts innerhalb eines beobachteten Raums erfasst, und ein Medizingerät oder Nicht-Medizingerät wird darüber angesteuert, so dass es selbst eine vorbestimmte Aktion ausführt.

Dokument US 2011/0037840 A1 zeigt ein Steuerungssystem und ein Verfahren, um eine Operationsleuchte zu betreiben. Das Steuerungssystem erkennt beispielsweise Hände, ggf. mit speziellen Markern, oder Köpfe der Operateure im Operationsfeld, und passt eine Beleuchtungssituation der Operationsleuchte an, um beispielsweise Schattenbildung zu verhindern oder reduzieren.

In Dokument US 2014/0177909 A1 ist eine drei-dimensionale interaktive Vorrichtung und ein Betriebsverfahren davon gezeigt. Die Vorrichtung enthält eine Projektionseinheit, eine Bilderfassungseinheit und eine Bildverarbeitungseinheit. Somit ist mittels der Vorrichtung und des Betriebsverfahrens eine Gestensteuerung von Geräten möglich.

Auch Dokument WO 2011/085813 A1 offenbart eine Gestenunterstützungseinrichtung zum Steuern oder Betreiben eines Medizingeräts. Durch ein Gestenerfassungssystem erfasste Gesten werden in eine Steuerung für das Medizingerät eingegeben, wobei einzelne, begrenzte Bereiche, in denen die Gesten ausgeführt werden, erkannt werden.

Die Erfindung wurde basierend auf der Aufgabe getätigt, die obigen Nachteile auszuräumen und eine Steuerungsvorrichtung für Medizingeräte bereitzustellen, die eine kontaktlose, einfache Bedienung der Medizingeräte erlaubt, so dass der Operateur selbst die Geräte bedienen kann, ohne unsteril zu werden.

Die Aufgabe wird durch eine Steuerungsvorrichtung gemäß Anspruch 1, ein System gemäß Anspruch 12 oder 13 sowie ein Verfahren zum Ansteuern von Medizingeräten gemäß Anspruch 15 gelöst.

Durch die erfindungsgemäße Steuerungsvorrichtung ist es möglich, ein Objekt, beispielsweise einen Finger eines Operateurs, sowie dessen Richtung zu erfassen. Über einen Sensor ist es möglich, zu erfassen auf welches eines zu aktivierenden Elements eines Medizinprodukts das Objekt gerichtet ist, wobei eine vorbestimmte Aktion des Elements ausgeführt wird. Dadurch ist eine kontaktlose Bedienung des Medizingeräts möglich und auch eine intuitive Bedienung des Medizingeräts wird ermöglicht.

Die Erfindung wird nun anhand von Ausführungsbeispielen unter Bezugnahme auf die beigefügten Zeichnungen erläutert.

Insbesondere zeigen
- Fig. 1: eine Operationsleuchte und einen Operationstisch als zwei Beispiele für Medizingeräte mit einem 3D-Sensor und einer erfindungsgemäßen Steuerungsvorrichtung,
- Fig. 2: verschiedene Bewegungen von Händen zum Ansteuern eines Medizingeräts,
- Fig. 3: eine Anwendung der Steuerungsvorrichtung, und
- Fig. 4: weitere Anwendungen der Steuerungsvorrichtung.

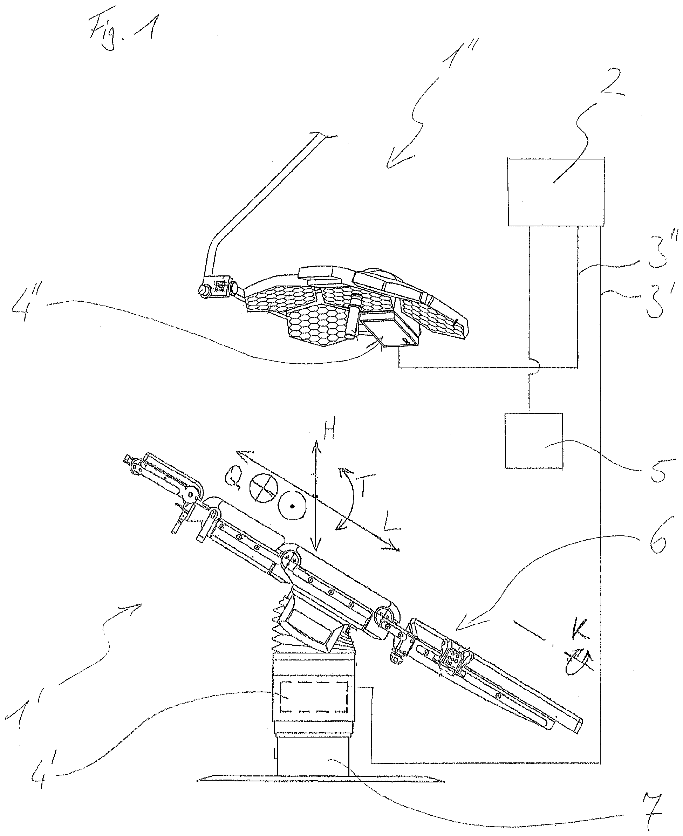

In Fig. 1 sind ein Operationstisch 1' und eine Operationsleuchte 1" als Beispiele für anzusteuernde Medizingeräte gezeigt. Der Operationstisch 1' und die Operationsleuchte 1" sind über Datenleitungen 3', 3" mit einer Steuerungsvorrichtung 2 verbunden. Genauer sind eine Steuerung 4' des Operationstischs 1' und eine Steuerung 4" der Operationsleuchte 1" über die Datenleitungen 3', 3" mit der Steuerungsvorrichtung 2.verbunden. Die Verbindung ist hier über die Datenleitungen 3', 3" realisiert, kann aber alternativ auch drahtlos über Funk oder Infrarot erfolgen. Ebenso kann die Steuerungsvorrichtung 2 alternativ in der Steuerung 4*'* des Operationstisches 1*'* oder der Steuerung 4*"* der Operationsleuchte 1*"*, also grundsätzlich in der Steuerung eines Medizingeräts, enthalten sein

Weiterhin ist ein 3D-Sensor 5 mit der Steuerungsvorrichtung 2 verbunden. Mittels des 3D-Sensors 5 werden Objekte im Raum, deren Form, Position und Bewegung erfasst. Unter Erfassen der Bewegung wird hier verstanden, dass sowohl eine Translation oder Rotation des gesamten Objekts in einem bestimmten Bewegungsablauf, eine Verformung durch eine Bewegung von bestimmten Abschnitten des Objekts, als auch eine Kombination der Translation oder Rotation und der Verformung des Objekts erfasst wird. In einer alternativen Ausführungsform sind mehrere 3D-Sensoren vorgesehen, um die Objekte aus verschiedenen Richtungen zu erfassen. Somit sind eine größere Zuverlässigkeit bei der Erfassung des Objekts und ein größerer Raum, in dem das Objekt erfasst werden kann, möglich. Optional ist der 3D-Sensor oder sind die 3D-Sensoren an einer Raumdecke befestigt, so dass der Einsatz hinsichtlich Störungen unkritischer ist. Alternativ ist der mindestens eine 3D-Sensor 5 an dem Operationstisch 1*'* oder der Operationsleuchte 1*"*, also an dem Medizingerät selbst, angebracht oder darin integriert. Die Verwendung eines 3D-Sensors stellt eine geeignete Sensorauswahl dar, wobei die Verwendung nicht auf einen 3D-Sensor beschränkt ist, sondern ein anderer geeigneter Sensor eingesetzt werden kann. Auch über den Einsatz von Kamerasystemen zur Bilderkennung kann die zur Steuerung beabsichtigte Geste erkannt werden.

In einer Ausführungsform ist der 3D-Sensor 5 eine sogenannte ToF (Time of Flight)-Kamera. Hier werden für einzelne Bildpunkte die Abstände von der Kamera so gemessen, dass das Objekt mit einem Lichtpuls ausgeleuchtet wird, wobei das Licht durch das Objekt reflektiert wird. Die Zeit, die das Licht ab dem Aussenden bis zur Rückkehr zu der ToF-Kamera benötigt, wird für jeden Bildpunkt gemessen, wobei die Zeit proportional zum Abstand ist. Dabei wird das Objekt nicht abgetastet, sondern das ganze Objekt gleichzeitig aufgenommen.

Die Steuerungsvorrichtung 2 ist dazu angepasst, die Bewegungen des Objekts in vorbestimmte Aktionen der Medizinprodukte umzuwandeln.

Die Aktionen der Operationsleuchte 1" sind beispielsweise eine Helligkeitsveränderung, eine Veränderung des Leuchtfelddurchmessers, eine Veränderung der Fokuslage, eine Veränderung einer Farbtemperatur des ausgesendeten Lichts oder eine Veränderung von aktiven Zonen, in denen Leuchtmittel der Operationsleuchte 1" betrieben werden.

Der Operationstisch 1' weist eine Patientenlagerungsplatte 6 und eine Säule 7 auf. In der Patientenlagerungsplatte 6 und in der Säule 7 sind mehrere Antriebe vorgesehen, mit denen die Patientenlagerungsplatte 6 verfahren werden kann.

Die Aktionen des Operationstischs 1' sind beispielsweise eine Längsverschiebung L, bei der die Patientenlagerungsplatte 7 entlang ihrer Längsachse verschoben wird, oder eine Querverschiebung Q, bei der die Patientenlagerungsplatte 7 quer zu ihrer Längsachse verschoben wird. Des Weiteren sind noch eine Kantung K, bei der die Patientenlagerungsplatte 7 um ihre Längsachse verschwenkt wird, und eine Trendelenburg- oder Antitrendelenburgverstellung möglich, bei der die Patientenlagerungsplatte 7 um ihre Querachse verschwenkt wird. Darüber hinaus besteht noch die Möglichkeit, die gesamte Patientenlagerungsplatte 7 über eine Höhenverstellung H in ihrer Höhe zu verändern. Schließlich können einzelne Segmente der Patientenlagerungsplatte 7 gegeneinander verschwenkt werden, um spezielle Lagerungen für bestimmte Eingriffe an einem Patienten zu realisieren.

Die Auswahl des anzusteuernden Medizingeräts aus mehreren Medizingeräten, hier also, ob eine Aktion des Operationstisches 1*'* oder der Operationsleuchte 1*"* ausgelöst werden soll, erfolgt über die Aktivierung des Medizingeräts über eine physische oder virtuelle Bedieneinheit über die Ausführung einer Geste in einem für das Medizingerät oder einer seiner Komponenten oder Funktionen vorgesehenen Erfassungsraum, über die Verwendung medizingeräteabhängiger Gesten und/oder eine gestenbasierte Selektion, wie sie später noch näher beschrieben wird.

Grundsätzlich können je nach anzusteuerndem Medizingerät und/oder ausgewählten Komponenten gleiche Gesten in unterschiedliche Steuerungsbefehle umgesetzt werden. Der einem Medizingerät, Komponenten und/oder Funktionen zugeordnete Erfassungsraum, kann auch nur für eine An-/Abmeldegeste relevant sein, während die Geste für den Steuerbefehl selbst dann wieder außerhalb dieses Raumes erfolgen kann, wenn dies vorteilhaft erscheint. Durch definierte Erfassungsräume kann aber auch auf eine An-/Abmeldegeste verzichtet werden, wenn der Erfassungsraum üblicherweise nicht ohne Ansteuerungsabsicht genutzt wird.

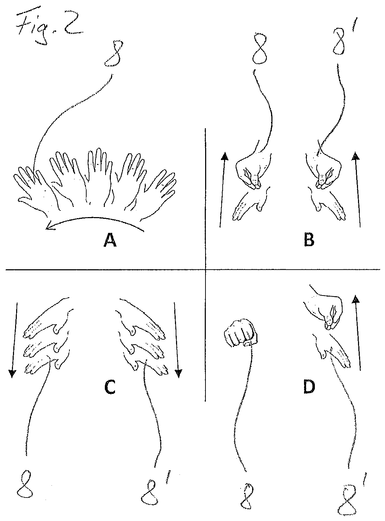

Fig. 2 zeigt beispielhaft vier Gesten, also Bewegungen von Händen als Objekte, deren Bewegungen durch die Steuerungsvorrichtung 2 in einen Steuerungsbefehl für eine bestimmte Aktion des Operationstischs 1' als Beispiel für das Medizinprodukt umgewandelt wird.

Darstellung A zeigt ein Winken mit einer einzelnen Hand 8. Die Hand 8 ist hier flach gehalten und wird während des Winkens, also während der Translation bzw. Rotation, nicht verformt, also beispielsweise nicht geballt.

Darstellung B zeigt eine Bewegung mit zwei Händen 8, 8', wobei die Hände 8, 8' sowohl eine Verformung, nämlich ausgehend von einer flachen Hand in eine Form, in der, angelehnt an ein Greifen, die Spitzen der Finger zusammengeführt werden, als auch eine Translation, nämlich von unten nach oben, ausführen. Der Bewegungsgrundsatz ist auch auf eine Einhandgeste direkt übertragbar.

In Darstellung C ist eine Bewegung gezeigt, in der beide Hände 8, 8' flach bleiben und von oben nach unten bewegt werden.

In Darstellung D ist eine Geste gezeigt, bei der eine der Hände 8 zu einer Faust geballt an einer Stelle bleibt, wohingegen die andere Hand 8', wie in Darstellung B beschrieben, verformt wird und dann von unten nach oben oder in einem Bogen um die Faust der anderen Hand bewegt wird.

Die Steuerungsvorrichtung 2 ist angepasst, eine spezielle der Gesten als Anmeldebewegung und als Abmeldebewegung zu verarbeiten. Hier zeigt Darstellung A die Anmeldebewegung als Autorisierungsgeste. Erst nachdem die Anmeldebewegung ausgeführt wurde, ist die Steuerungsvorrichtung 2 angepasst, weitere Bewegungen der Hand oder der Hände so zu verarbeiten, dass sie in Steuerungsbefehle für den Operationstisch 1' umgewandelt werden.

Nach einem Ausführen der gewünschten Bewegung wird dann wiederum die Anmeldebewegung ausgeführt, wobei die Steuerungsvorrichtung 2 so angepasst ist, dass diese Bewegung dann als Abmeldebewegung verstanden wird, so dass keine weiteren Bewegungen von der Steuerungsvorrichtung in einen Steuerungsbefehl für eine Aktion des Operationstischs 1' umgewandelt werden. Lediglich eine erneute Anmeldebewegung wird wiederum verarbeitet.

In der Darstellung B ist die Geste dargestellt, mit der in dieser Ausführungsform des Operationstischs 1' die Höhenverstellung H aktiviert wird, und die Patientenlagerungsplatte 7 nach oben bewegt wird. Die Verstellung ist für den gesamten Tisch möglich, kann aber auch nur für ausgewählte Segmente, bspw. durch eine Gestenausführung in einem dem Segment zugeordneten Erfassungsraum, vorgesehen sein. Bei der Höhenverstellung wird die Geste von mehreren Händen 8, 8' ausgeführt, die eine vorbestimmte Bewegung, nämlich die Verformung, nämlich ausgehend von einer flachen Hand in eine Form, in der die Spitzen der Finger zusammengeführt werden, und anschließende Translation der Hände von unten nach oben ausführen. Die Steuerungsvorrichtung 2 ist angepasst, zu erkennen, ob mehrere Hände 8, 8' die vorbestimmte Bewegung ausführen und wandelt die erfassten Bewegungen entsprechend einer Kombination der erfassten Bewegungen in die vorbestimmte Aktion, hier die Bewegung der Patientenlagerungsplatte 7 nach oben, um. Eine Einhandgeste mit gleichem oder alternativem Gestenmuster ist ebenfalls denkbar.

Auch in den Darstellungen C und D wird die Geste von mehreren Händen 8, 8' ausgeführt, wobei in Darstellung C beide Hände in einer bloßen Translation flach nach unten bewegt werden, so dass die Höhenverstellung H aktiviert wird und die Patientenlagerungsplatte 7 nach unten verstellt wird. In der Darstellung D ist die eine Hand 8 aber nicht translatorisch bewegt oder verformt, sondern lediglich die Form, also die Hand 8 als eine Faust, wird erfasst. Die andere Hand 8' führt sowohl eine Translation als auch eine Verformung der Hand durch. Dadurch wird eine Trendelenburg-Verstellung bzw. eine Antitrendelenburg-Verstellung angesteuert.

Die Bewegung sowie die Verformung der Hand 8, 8' werden dann erfasst, wenn sie mit einer bestimmten Geschwindigkeit innerhalb eines vorbestimmten Toleranzbereichs ausgeführt werden. Wenn eine Bewegung zu schnell oder zu langsam ausgeführt wird, wird sie nicht als vorbestimmte Bewegung erfasst, sondern ignoriert.

Die Geschwindigkeit eines Bewegungsbefehls kann grundsätzlich der Geschwindigkeit der Gestenbewegung entsprechen oder zu dieser in einem definierten Verhältnis stehen, einem Objektabstand oder einer Ebene im Erfassungsraum entsprechen und/oder von einer Zusatzgeste abhängig sein.

Die vorbestimmten Aktionen des Operationstischs 1' werden optional in Echtzeit ausgeführt, was bedeutet, dass die Steuerungsvorrichtung 2 so angepasst ist, dass die erfassten Bewegungen unmittelbar von dem Operationstisch 1' ausgeführt werden.

Neben einer Direktsteuerung des Medizingeräts kann der Benutzer dieses alternativ auch über ein Modell steuern. Das Modell des Medizingeräts wird auf einer Benutzeroberfläche bzw. einer Wiedergabeeinheit wie einem Monitor dargestellt. Wird das Modell über Bewegungsbefehle angesteuert, bewegt sich das Medizingerät selbst analog dazu. Die Position und/oder der jeweilige Bewegungs- bzw. Ansteuerungsbefehl, sofern nicht eine Bewegung, sondern eine anderweitige Aktion initiiert werden soll, ist über einen Cursor, ggf. in unterschiedlicher Erscheinungsform in Abhängigkeit des Befehls, auf der Wiedergabeeinheit nachvollziehbar. Hierdurch erhöht sich die sichere Bedienung durch die visuelle Nachvollziehbarkeit der Benutzerhandlung. Zudem kann die Bedienung unter Beibehaltung der visuellen Befehlskontrolle räumlich vom zu bedienenden Medizingerät getrennt werden.

Die Zuordnung der Gesten und der vorbestimmten Aktionen des Medizinprodukts sind in alternativen Ausführungsformen verschieden von dieser Ausführungsform. Es besteht ferner die Möglichkeit zusätzlich oder alternativ auch andere Gesten oder Gesten von anderen Objekten, beispielsweise von Beinen zu erfassen.

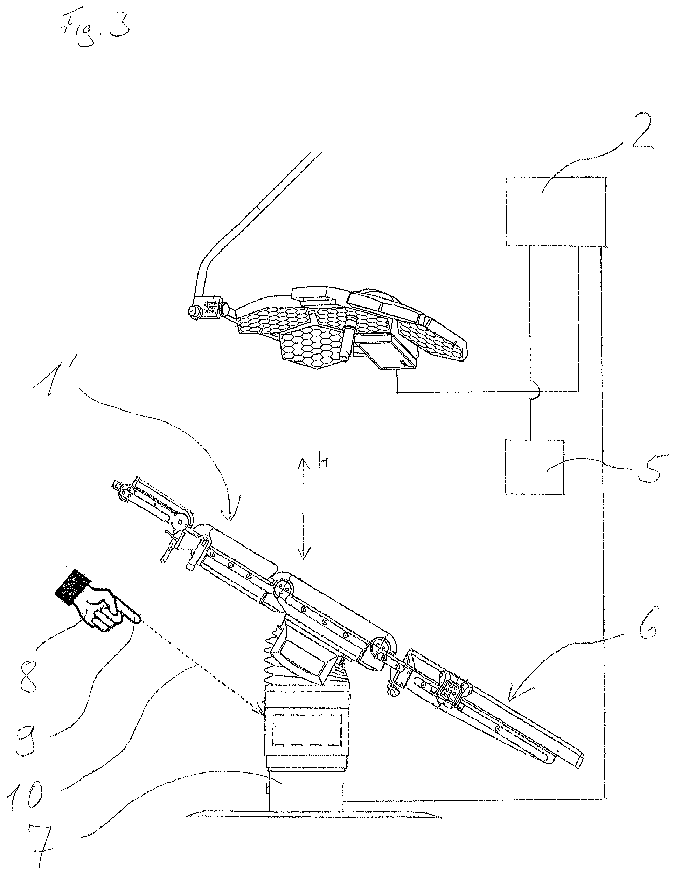

In Fig. 3 ist eine Alternative oder eine Ergänzung einer Ansteuerung des Operationstischs 1' gezeigt. Auch hier wird eine Bewegung der Hand 8 mittels des 3D-Sensors 5 oder des anderen Sensors erfasst und an die Steuerungsvorrichtung 2 weitergegeben. Im Speziellen wird hier die Bewegung in Verbindung mit einer Ausrichtung eines ausgestreckten Fingers 9 erfasst. Die Hand 8 ist in Verbindung mit dem ausgestreckten Finger 9 als stabartig anzusehen. Dies bedeutet, dass es möglich ist, die Ausrichtung der Hand 8 mit dem ausgestreckten Finger 9 zu erfassen. Die Ausrichtung wird mittels erfasster Punkte des Fingers 9 als eine Achse des Fingers bestimmt. Ein Vektor 10 wird als eine Verlängerung der Verbindung dieser Punkte bestimmt. Alternativ werden andere Punkte der Hand 8 und des Fingers 9 zur Bestimmung des Vektors 10 verwendet. Wesentlich ist, dass über die Punkte eine eindeutige Vektorbestimmung möglich ist. In weiteren Alternativen wird der Vektor 10 aus der Orientierung eines anderen stabartigen Objekts bestimmt, der beispielsweise in der Hand 8 gehalten wird.

Der 3D-Sensor 5 erfasst in dieser Ausführungsform mehrere Elemente des Operationstischs 1', die ansteuerbar, also zu aktivieren sind. Hier ist das Element exemplarisch der Antrieb für die Höhenverstellung in der Säule 7. Die Steuerungsvorrichtung 2 erkennt aus der Richtung des Vektors 10 und einer Lage der Hand 8, somit aus dem von der Hand 8 ausgehenden Vektor 10, dass es einen Schnittpunkt des Vektors 10 und der Säule 7 gibt, und erkennt durch die Position des Schnittpunkts das zu aktivierende Element, das eine vorbestimmte Aktion auszuführen hat. Die vorbestimmte Aktion wird dann, wie vorangehend beschrieben, durch erfasste vorbestimmte Bewegungen und/oder die Geste und/oder den einer Funktionalität/einem Steuerungsbefehl zugeordneten Erfassungsraum der Hand 8 oder der Hände 8, 8' ausgeführt. In einer alternativen Ausführungsform ist die Steuerungsvorrichtung 2 angepasst, die vorbestimmte Aktion über das Erfassen einer andersartigen Anweisung als einer Geste, beispielsweise über eine Spracheingabe, auszuführen.

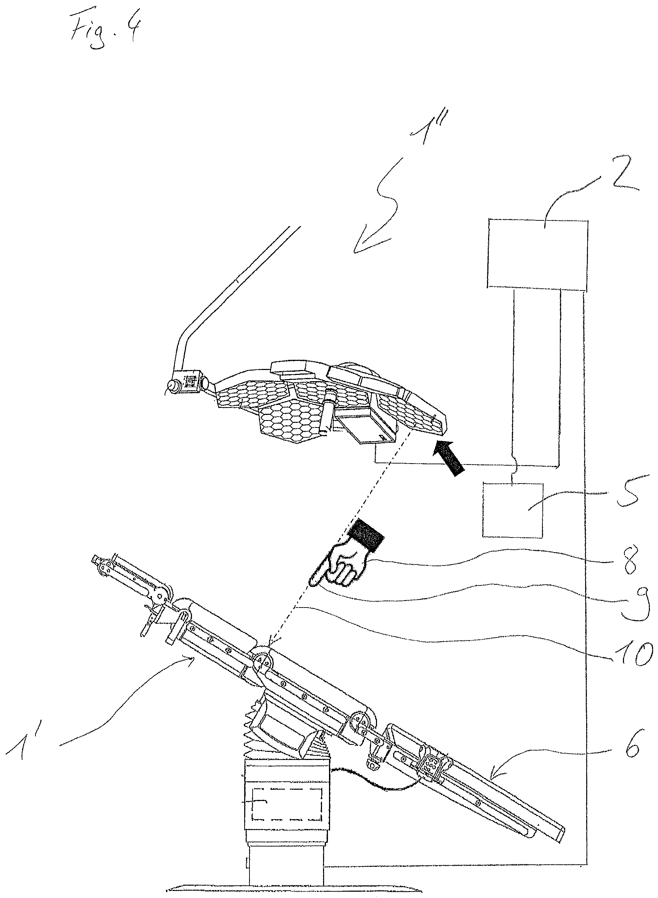

In Fig. 4 ist eine weitere Möglichkeit der Ansteuerung, hier anhand der Operationsleuchte 1" gezeigt.

Die Steuerungsvorrichtung 2 ist hier so ausgebildet, dass der Vektor 10 wiederum über die Punkte an dem Finger 9 und dessen Achse bestimmt wird. Die Steuerungsvorrichtung 2 erkennt über die Erfassung der Punkte durch den 3D-Sensor 5 die Richtung des Vektors 10. Ferner erfasst die Steuerungsvorrichtung 2 über den 3D-Sensor menschliche Körper, die sich in seinem Erfassungsbereich befinden.

Auch hier wird ein Schnittpunkt des Vektors 10 mit dem menschlichen Körper (nicht gezeigt) erfasst. Die Steuerungsvorrichtung 2 ist so angepasst, dass sie eine zuvor ausgewählte Aktion bezüglich der Stelle des menschlichen Körpers ausführt. In Fig. 4 ist gezeigt, dass der Vektor auf eine Stelle auf der Patientenlagerungsplatte 6 gerichtet ist. Die zuvor gewählte Aktion, ausgewählt beispielsweise über das Zeigen auf die Operationsleuchte 1", wird dann so ausgeführt, dass die Stelle auf der Patientenlagerungsplatte 6, auf der der menschliche Körper liegt, beleuchtet wird. Dies erfolgt beispielsweise über ein motorisches Verstellen von Modulen der Operationsleuchte 1" oder über das Aktivieren von auf die Stelle gerichteten Leuchtmitteln.

In Fig. 4 ist des Weiteren eine Funktion einer optionalen Anpassung der Steuerungsvorrichtung 2 gezeigt. Die räumliche Orientierung des Vektors 10 bestimmt zusätzlich zu dem Schnittpunkt mit dem menschlichen Körper in der Richtung des Vektors 10 auch einen zweiten Schnittpunkt mit einem zu aktivierenden Element des Medizingeräts in der entgegengesetzten Richtung des Vektors. Die zuvor ausgewählte Aktion, hier das Beleuchten der Stelle auf der Patientenlagerungsplatte 6 wird dann durch das Objekt, auf dem der Schnittpunkt des Vektors 10 mit dem Objekt liegt, in der Richtung der räumlichen Orientierung des Vektors 10 ausgeführt. Hier ist gezeigt, dass die Stelle auf der Patientenlagerungsfläche 6 durch das mit einem Pfeil gekennzeichnete Modul der Operationsleuchte 1" beleuchtet wird.

Die Verwendung des Vektors 10 kann zudem genutzt werden, um einen Schnittpunkt mit einer Oberfläche eines Zielbereichs, beispielsweise der Patientenauflage des Operationstisches bzw. dem darauf befindlichen Patienten, zu bilden. Kann dieser Schnittpunkt gebildet werden, so ist die Richtungsangabe mit einer damit zu verbindenden Funktionalität im ersten Ansatz plausibel und wird umgesetzt. Schlägt die Plausibilitätsprüfung fehl, weil beispielsweise eine Selektion ins Leere geht, so wird der Ansteuerungsbefehl nicht umgesetzt oder muss zunächst explizit durch den Benutzer bestätigt werden.

Die dem Operationstisch 1' und der Operationsleuchte 1" zugeordnet beschriebenen Funktionen können sinngemäß durch alle geeigneten mit der Steuerungsvorrichtung 2 verbundenen Medizingeräte ausgeführt werden. Die verschiedenen Ausführungsformen sind miteinander kombinierbar.

Im Betrieb erfasst die Steuerungsvorrichtung 2 über den 3D-Sensor 5 zunächst, ob die vorbestimmte Anmeldebewegung ausgeführt wird.

Im Anschluss an die vorbestimmte Anmeldebewegung wird die Bewegung des Objekts, also der Hand 8, 8' oder des Arms durch den 3D-Sensor erfasst und die vorbestimmte, der vorbestimmten Bewegung des Objekts zugeordnete, Aktion des Medizingeräts durch die Steuerungsvorrichtung 2 angesteuert.

Nach einem Beenden der gewünschten vorbestimmten Aktion des Medizingeräts wird zum Beenden der Bedienung die Abmeldebewegung ausgeführt, so dass keine weiteren Bewegungen nach der Abmeldebewegung als auszuführende Aktionen interpretiert werden.

Optional werden nicht nur die Bewegung von einem der Objekte, sondern von mehreren Objekten erfasst. Hier wird die Position und die Bewegung von beiden Händen 8, 8' erfasst und die vorbestimmte Aktion entsprechend der Kombination der Bewegungen der mehreren Objekte ausgeführt.

Bei der optionalen Funktion, bei der es möglich ist, das zu aktivierende Element des Medizinprodukts auszuwählen, wird nach dem Aktivieren und vor dem Ausführen der Bewegung zur Ansteuerung das gewünschte zu aktivierende Element durch Zeigen mittels des Fingers 9 (Richten des stabartigen Objekts) auf dieses Element ausgewählt. Dann wird die vorbestimmte Bewegung durch die Objekte ausgeführt und die Steuerungsvorrichtung 2 steuert das gewünschte zu aktivierende Element an, so dass die der vorbestimmten Bewegung entsprechende Aktion ausgeführt wird.

Bei der weiteren optionalen Funktion, in der die Stelle ausgewählt wird, auf die die Aktion des Elements des Medizingeräts gerichtet ist, wird nach einem optional erforderlichen Aktivieren des Elements, das die Aktion ausführt, mit dem Finger 9 auf die Stelle gezeigt, bezüglich der die Aktion ausgeführt werden soll. Dadurch steuert die Steuerungsvorrichtung 2 das gewünschte Element so an, dass die Aktion bezüglich der Stelle ausgeführt wird, also hier die Stelle auf der Patientenlagerungsfläche 6 beleuchtet wird.

Bei der weiteren optionalen Funktion, dass durch die Richtung, aus der gezeigt wird, auch das Element aktiviert wird, das die Aktion ausführen soll, wird zusätzlich zu der Stelle, bezüglich der die Aktion auszuführen ist, die Richtung des Fingers 9 (des stabartigen Objekts) erfasst und das bezüglich der Richtung des Fingers entgegengesetzte zu aktivierende Element, das die Aktion ausführen soll, ausgewählt. Das zu aktivierende Element wird dann angesteuert, so dass die Aktion aus der Richtung des Fingers 9 ausgeführt wird.

## Patentansprüche

1. Steuerungsvorrichtung (2) für ein Medizingerät, mit mindestens einem Sensor (5) zum dreidimensionalen Erfassen eines Objekts (8), wobei das Objekt (8) ausgebildet ist, seine Lage und, durch seine Ausrichtung, einen Vektor (10) für eine Richtung erfassbar zu machen, wobei
das Objekt (8) auf einen Zielbereich gerichtet ist, und
der von dem Objekt (8) ausgehende Vektor (10) mit einer Oberfläche des Zielbereichs einen Schnittpunkt bildet,
die Steuerungsvorrichtung (2) angepasst ist, zu erkennen, in welchem Zielbereich der Schnittpunkt liegt und eine für den Zielbereich vorbestimmte Aktion durch das Medizingerät zu veranlassen,
wobei die Steuerungsvorrichtung (2) dazu angepasst ist,
eine vorbestimmte Anmeldebewegung des Objekts (8, 8') so zu verarbeiten, dass die weiteren vorbestimmten Bewegungen des Objekts (8, 8') in den Steuerungsbefehl für die vorbestimmte Aktion des Medizingeräts umgewandelt werden, und
eine vorbestimmte Abmeldebewegung des Objekts (8, 8') so zu verarbeiten, dass keine weiteren Bewegungen des Objekts (8, 8') in einen Steuerungsbefehl für eine Aktion des Medizingeräts umgewandelt werden,
**dadurch gekennzeichnet, dass**
die Steuerungsvorrichtung (2) dazu angepasst ist, eine spezielle von Gesten als die vorbestimmte Anmeldebewegung und die vorbestimmte Abmeldebewegung zu verarbeiten, wobei Gesten Bewegungen von Händen als Objekte sind.

2. Steuerungsvorrichtung (2) gemäß Anspruch 1, wobei
die Steuerungsvorrichtung (2) angepasst ist, die vorbestimmte Aktion bezüglich des Zielbereichs nur auszuführen, wenn eine Plausibilitätsprüfung des Schnittpunkts bestanden ist, oder die vorbestimmte Aktion auf Grund des durch das Objekt (8) und dessen Richtung bestimmten Vektors (10) auszuführen, wenn eine zusätzliche Bestätigung vorliegt.

3. Steuerungsvorrichtung (2) gemäß Anspruch 1 oder 2, wobei
das Objekt stabartig ausgebildet ist oder zumindest einen stabartigen Abschnitt aufweist, der ein einziges freies Ende aufweist, und die Steuerungsvorrichtung (2) dazu angepasst ist, eine Richtung durch das freie Ende und eine Achse des stabartigen Objekts zu bestimmen.

4. Steuerungsvorrichtung gemäß einem der vorangehenden Ansprüche, wobei
der mindestens eine Sensor (5) ausgebildet ist, einen menschlichen Körper zu erkennen, und
die Steuerungsvorrichtung (2) angepasst ist, zu erkennen, auf welche Stelle des menschlichen Körpers das Objekt (8) gerichtet ist, und die vorbestimmte Aktion bezüglich der Stelle des menschlichen Körpers zu veranlassen.

5. Steuerungsvorrichtung gemäß einem der vorangehenden Ansprüche, wobei
der mindestens eine Sensor (5) ausgebildet ist, zu aktivierende Elemente des Medizinprodukts zu erkennen, und
die Steuerungsvorrichtung (2) angepasst ist, zu erkennen, auf welche zu aktivierenden Elemente das Objekt (8) gerichtet ist, und die vorbestimmte Aktion bezüglich der erkannten zu aktivierenden Elemente zu veranlassen.

6. Steuerungsvorrichtung (2) gemäß einem der Ansprüche 1 bis 4, wobei
die Steuerungsvorrichtung (2) angepasst ist, aus einer räumlichen Orientierung des Objekts (8) und dessen Richtung eine Richtung der vorbestimmten Aktion zu bestimmen und das Medizingerät so anzusteuern, dass die vorbestimmte Aktion durch das entgegengesetzt zu der Richtung liegende zu aktivierende Element ausgeführt wird.

7. Steuerungsvorrichtung (2) gemäß einem der vorangehenden Ansprüche, wobei
der mindestens eine Sensor (5) zusätzlich zum Erfassen einer Bewegung des Objekts (8, 8') ausgebildet ist, und
die Steuerungsvorrichtung (2) angepasst ist,
eine von dem mindestens einen Sensor (5) erfasste vorbestimmte Bewegung des mindestens einen Objekts (8, 8') in einen Steuerungsbefehl für die vorbestimmte Aktion des Medizingeräts umzuwandeln.

8. Steuerungsvorrichtung (2) gemäß einem der vorangehenden Ansprüche, wobei der mindestens eine Sensor (5) eine TOF-Kamera ist, die dazu angepasst ist, das Objekt (8, 8') über eine Laufzeit von ausgesendetem und reflektiertem Licht zu erfassen.

9. Steuerungsvorrichtung (2) gemäß einem der vorangehenden Ansprüche, wobei die Steuerungsvorrichtung (2) angepasst ist, zu erkennen, ob mehrere Objekte (8, 8') eine jeweils vorbestimmte Bewegung ausführen, und den vorbestimmten Steuerungsbefehl für die vorbestimmte Aktion des Medizingeräts entsprechend einer Kombination der jeweiligen Bewegung der mehreren Objekte (8, 8') umzuwandeln.

10. Steuerungsvorrichtung (2) gemäß einem der vorangehenden Ansprüche, wobei die Steuerungsvorrichtung (2) angepasst ist, die vorbestimmte Aktion in Echtzeit auszuführen.

11. Steuerungsvorrichtung gemäß einem der vorangehenden Ansprüche, wobei mehrere Sensoren vorgesehen sind.

12. System aus einem Operationstisch (1') und einer Steuerungsvorrichtung (2) gemäß einem der Ansprüche 1 bis 11.

13. System aus einer Operationsleuchte (1") und einer Steuerungsvorrichtung (2) gemäß einem der Ansprüche 1 bis 11.

14. System aus einer Operationsleuchte(1") und einer Steuerungsvorrichtung (2) gemäß Anspruch 13, wobei
die vorbestimmte Aktion eine Veränderung einer Fokuslage ist, so dass ein Fokuspunkt von Lichtstrahlen der Operationsleuchte auf der Oberfläche des Zielbereichs liegt.

15. Verfahren zum Ansteuern eines Medizingeräts mit einer Steuerungsvorrichtung (2) gemäß einem der Ansprüche 1 bis 11, wobei das Verfahren die folgenden Schritte aufweist:
- Ausführen der speziellen der Gesten als Anmeldebewegung,
- Erfassen des Vektors (10) und der Oberfläche von Zielbereichen durch den mindestens einen Sensor (5),
- Bestimmen des Schnittpunkts des Vektors (10) und des Zielbereichs, auf den das Objekt (8) gerichtet ist,
- Ansteuern der für den Zielbereich vorbestimmten Aktion des Medizingeräts, und
- Ausführen der speziellen der Gesten als Abmeldebewegung, so dass keine weiteren Bewegungen von der Steuerungsvorrichtung (2) in einen Steuerungsbefehl für eine Aktion des Medizingeräts umgewandelt werden, bis eine erneute Anmeldebewegung verarbeitet wird.

16. Verfahren zum Ansteuern eines Medizingeräts gemäß Anspruch 15 mit einer Steuerungsvorrichtung (2) gemäß Anspruch 5 oder einem der Ansprüche 5 und 7 bis 11, wobei das Verfahren die folgenden Schritte aufweist:
- Erfassen der zu aktivierenden Elemente des Medizingeräts durch den Sensor (5),
- Erfassen durch den mindestens einen Sensor (5), auf welches zu aktivierende Element des Medizingeräts das Objekt (8) gerichtet ist, und
- Ansteuern der vorbestimmten Aktion des zu aktivierenden Elements, auf das das Objekt (8) gerichtet ist.

17. Verfahren zum Ansteuern eines Medizingeräts gemäß Anspruch 15 mit einer Steuerungsvorrichtung (2) gemäß Anspruch 2 oder einem der Ansprüche 2 und 3 bis 11, wobei
die Steuerungsvorrichtung (2) die vorbestimmte Aktion bezüglich des Zielbereichs nur ausführt, wenn die Plausibilitätsprüfung des Schnittpunkts bestanden ist, oder die vorbestimmte Aktion auf Grund des durch das Objekt (8) und dessen Richtung bestimmten Vektors (10) ausführt, wenn eine zusätzliche Bestätigung vorliegt.

18. Verfahren zum Ansteuern eines Medizingeräts gemäß Anspruch 15 mit einer Steuerungsvorrichtung (2) gemäß Anspruch 6 oder einem der Ansprüche 6 und 7 bis 11, wobei
das Verfahren die folgenden Schritte aufweist:
- Erfassen einer Richtung des Objekts aus seiner Orientierung und seines Endes,
- Erfassen des zu aktivierenden Elements des Medizingeräts, das sich entgegengesetzt zu der Richtung des Elements (8) befindet, und
- Ansteuern des zu aktivierenden Elements, das die vorbestimmte Aktion aus der Richtung des Objekts (8) ausführt.

## Claims

1. A control apparatus (2) for a medical device, having at least one sensor (5) for three-dimensional detection of an object (8), the object (8) being designed to allow its position and, by its orientation, a vector (10) for a direction to be detected, where
the object (8) is directed at a target area, and
the vector (10) originating from the object (8) forms a point of intersection with a surface of the target area,
the control apparatus (2) is configured to detect in which target area the point of intersection lies and to trigger an action by the medical device, which action has been predetermined for that target area,
where the control apparatus (2) is configured
to process a predetermined registration movement of the object (8, 8') such that the further predetermined movements of the object (8, 8') are converted into the control command for the predetermined action of the medical device, and
to process a predetermined deregistration movement of the object (8, 8') such that no further movements of the object (8, 8') are converted into a control command for an action of the medical device,
wherein
the control apparatus (2) is configured to process specific gestures as the predetermined registration movement and the predetermined deregistration movement, with gestures being movements of hands as objects.

2. The control apparatus (2) of Claim 1, wherein
the control apparatus (2) is configured to execute the predetermined action with regard to the target area only if a plausibility check on the point of intersection is passed, or to execute the predetermined action on the basis of the vector (10) determined by the object (8) and its direction if there is additional confirmation.

3. The control apparatus (2) of Claim 1 or 2, wherein
the object is designed to be rod-like or to have at least one rod-like section comprising one single free end, and the control apparatus (2) is configured to determine a direction through the free end and an axis of the rod-like object.

4. The control apparatus of one of the preceding claims, wherein
the at least one sensor (5) is configured to detect a human body, and
the control apparatus (2) is configured to detect at what location on the human body the object (8) is directed, and to trigger the predetermined action with regard to the location on the human body.

5. The control apparatus of one of the preceding claims, wherein
the at least one sensor (5) is configured to detect the elements of the medical product to be activated, and
the control apparatus (2) is configured to detect at which element to be activated the object (8) is directed, and to trigger the predetermined action with regard to the detected elements to be activated.

6. The control apparatus (2) of one of Claims 1 to 4, wherein
the control apparatus (2) is configured to determine a direction of the predetermined action from a spatial orientation of the object (8) and its direction and to actuate the medical device such that the predetermined action is executed by the element to be activated that lies opposite to that direction.

7. The control apparatus (2) of one of the preceding claims, wherein
the at least one sensor (5) is also designed to detect a movement of the object (8, 8') and
the control apparatus (2) is configured
to convert a predetermined movement of the at least one object (8, 8') detected by the at least one sensor (5) into a control command for the predetermined action of the medical device.

8. The control apparatus (2) of one of the preceding claims, wherein the at least one sensor (5) is a ToF camera, which is configured to detect the object (8, 8') on the basis of the travel time of emitted and reflected light.

9. The control apparatus (2) of one of the preceding claims, wherein the control apparatus (2) is configured to detect whether multiple objects (8, 8') are each carrying out a predetermined movement, and to convert the predetermined control command for the predetermined action of the medical device according to a combination of each movement of the multiple objects (8, 8').

10. The control apparatus (2) of one of the preceding claims, wherein the control apparatus (2) is configured to execute the predetermined action in real-time.

11. The control apparatus of one of the preceding claims, wherein multiple sensors are provided.

12. A system comprising an operating table (1') and the control apparatus (2) of one of Claims 1 to 11.

13. A system comprising a surgical light (1") and the control apparatus (2) of one of Claims 1 to 11.

14. The system comprising a surgical light (1") and the control apparatus (2) of Claim 13, wherein
the predetermined action is a change of focus position, such that a focal point of rays of light from the surgical light lies on the surface of the target area.

15. A method for actuating a medical device using the control apparatus (2) of one of Claims 1 to 11, wherein the method comprises the following steps:
- executing the specific gestures as a registration movement,
- detecting, via the at least one sensor (5), the vector (10) and the surface of target areas,
- determining the point of intersection of the vector (10) and the target area at which the object (8) is directed,
- actuating the action of the medical device, which action has been predetermined for the target area, and
- executing the specific gestures as a deregistration movement, such that no further movements are converted by the control apparatus (2) into a control command for an action of the medical device until a new registration movement is processed.

16. The method for actuating a medical device of Claim 15 using the control apparatus (2) of Claim 5 or one of Claims 5 and 7 to 11, wherein the method comprises the following steps:
- detecting, via the sensor (5), the elements of the medical device to be activated,
- detecting, via the at least one sensor (5), at which element of the medical device to be activated the object (8) is directed, and
- actuating the predetermined action of the element to be activated at which the object (8) is directed.

17. The method for actuating a medical device of Claim 15 using the control apparatus (2) of Claim 2 or one of Claims 2 and 3 to 11, wherein
the control apparatus (2) executes the predetermined action with regard to the target area only if the plausibility check on the point of intersection is passed, or executes the predetermined action on the basis of the vector (10) determined by the object (8) and its direction if there is additional confirmation.

18. The method for actuating a medical device of Claim 15 using the control apparatus (2) of Claim 6 or one of Claims 6 and 7 to 11, wherein the method comprises the following steps:
- detecting a direction of the object from its orientation and its end,
- detecting the element of the medical device to be activated, which lies opposite to the direction of the element (8), and
- actuating the element to be activated, which executes the action that has been predetermined from the direction of the object (8).

## Revendications

1. Appareil de commande (2) pour un dispositif médical, comportant au moins un capteur (5) permettant une détection tridimensionnelle d'un objet (8), l'objet (8) étant conçu pour prévoir sa position et, au moyen de son orientation, un vecteur (10) permettant la détection d'une direction,
l'objet (8) étant dirigé vers une zone cible, et
le vecteur (10) tirant son origine de l'objet (8) formant un point d'intersection avec une surface de la zone cible,
l'appareil de commande (2) étant configuré pour détecter la zone cible dans laquelle se trouve le point d'intersection et pour déclencher une action par le dispositif médical,
laquelle action a été prédéfinie pour cette zone cible,
l'appareil de commande (2) étant configuré
pour traiter un mouvement d'enregistrement prédéfini de l'objet (8, 8') de sorte que les autres mouvements prédéfinis de l'objet (8, 8') soient convertis en l'instruction de commande de l'action prédéfinie du dispositif médical, et
pour traiter un mouvement de suppression d'enregistrement prédéfini de l'objet (8, 8') de sorte qu'aucun autre mouvement de l'objet (8, 8') ne soit converti en instruction de commande d'une action du dispositif médical,
dans lequel
l'appareil de commande (2) est configuré pour traiter des gestes spécifiques tels que le mouvement d'enregistrement prédéfini et le mouvement de suppression d'enregistrement prédéfini, les gestes étant des mouvements des mains en tant qu'objets.

2. Appareil de commande (2) selon la revendication 1, dans lequel
l'appareil de commande (2) est configuré pour exécuter l'action prédéfinie par rapport à la zone cible uniquement si un contrôle de plausibilité au point d'intersection est effectué, ou pour exécuter l'action prédéfinie sur la base du vecteur (10) déterminé par l'objet (8) et sa direction en cas de confirmation supplémentaire.

3. Appareil de commande (2) selon la revendication 1 ou 2, dans lequel
l'objet est conçu pour être de type tige ou pour avoir au moins une section de type tige comprenant une seule extrémité libre, et l'appareil de commande (2) est configuré pour déterminer une direction à travers l'extrémité libre et un axe de l'objet de type tige.

4. Appareil de commande selon l'une des revendications précédentes, dans lequel
le ou les capteurs (5) sont configurés pour détecter un corps humain, et
l'appareil de commande (2) est configuré pour détecter à quel endroit, sur le corps humain, l'objet (8) est dirigé et pour déclencher l'action prédéfinie par rapport à l'emplacement sur le corps humain.

5. Appareil de commande selon l'une des revendications précédentes, dans lequel
le ou les capteurs (5) sont configurés pour détecter les éléments du produit médical à activer, et
l'appareil de commande (2) est configuré pour détecter vers quel élément devant être activé l'objet (8) est dirigé, et pour déclencher l'action prédéfinie par rapport aux éléments détectés à activer.

6. Appareil de commande (2) selon l'une des revendications 1 à 4, dans lequel
l'appareil de commande (2) est configuré pour déterminer une direction de l'action prédéfinie à partir d'une orientation spatiale de l'objet (8) et de sa direction et pour actionner le dispositif médical de sorte que l'action prédéfinie soit exécutée par l'élément à activer qui se trouve à l'opposé de cette direction.

7. Appareil de commande (2) selon l'une des revendications précédentes, dans lequel
le ou les capteurs (5) sont également conçus pour détecter un mouvement de l'objet (8, 8') et
l'appareil de commande (2) est configuré
pour convertir un mouvement prédéfini du ou des objets (8, 8') détectés par le ou les capteurs (5) en une instruction de commande destinée à l'action prédéfinie du dispositif médical.

8. Appareil de commande (2) selon l'une des revendications précédentes, dans lequel le ou les capteurs (5) sont une caméra ToF, qui est configurée pour détecter l'objet (8, 8') sur la base du temps de déplacement de la lumière émise et réfléchie.

9. Appareil de commande (2) selon l'une des revendications précédentes, dans lequel l'appareil de commande (2) est configuré pour détecter si plusieurs objets (8, 8') effectuent chacun un mouvement prédéfini, et pour convertir l'instruction de commande prédéfinie de l'action prédéfinie du dispositif médical conformément à une combinaison de chaque mouvement des objets multiples (8, 8').

10. Appareil de commande (2) selon l'une des revendications précédentes, dans lequel l'appareil de commande (2) est configuré pour exécuter l'action prédéfinie en temps réel.

11. Appareil de commande selon l'une des revendications précédentes, dans lequel plusieurs capteurs sont fournis.

12. Système comprenant une table d'opération (1') et l'appareil de commande (2) selon l'une des revendications 1 à 11.

13. Système comprenant une lampe chirurgicale (1") et l'appareil de commande (2) selon l'une des revendications 1 à 11.

14. Système comprenant une lampe chirurgicale (1") et l'appareil de commande (2) selon la revendication 13, dans lequel
l'action prédéfinie représente un changement de position de mise au point, de sorte qu'un point focal des rayons de lumière émanant de la lampe chirurgicale se trouve sur la surface de la zone cible.

15. Procédé d'actionnement d'un dispositif médical faisant appel à des appareils de commande (2) selon l'une des revendications 1 à 11, dans lequel le procédé comprend les étapes suivantes consistant à :
- exécuter les gestes spécifiques en tant que mouvement d'enregistrement,
- détecter, par l'intermédiaire du ou des capteurs (5), le vecteur (10) et la surface des zones cibles,
- déterminer le point d'intersection du vecteur (10) et la zone cible vers laquelle l'objet (8) est dirigé,
- déclencher l'action du dispositif médical, laquelle action a été prédéfinie pour la zone cible, et
- exécuter les gestes spécifiques en tant qu'un mouvement de suppression d'enregistrement, de sorte qu'aucun autre mouvement n'est converti par l'appareil de commande (2) en une instruction de commande pour une action du dispositif médical jusqu'à ce qu'un nouveau mouvement d'enregistrement soit traité.

16. Procédé d'actionnement d'un dispositif médical selon la revendication 15 faisant appel à l'appareil de commande (2) selon la revendication 5 ou l'une des revendications 5 et 7 à 11, dans lequel le procédé comprend les étapes suivantes consistant à :
- détecter, par l'intermédiaire du capteur (5), les éléments du dispositif médical à activer,
- détecter, par l'intermédiaire du ou des capteurs (5), vers quel élément du dispositif médical à activer l'objet (8) est dirigé, et
- déclencher l'action prédéfinie de l'élément à activer vers lequel l'objet (8) est dirigé.

17. Procédé d'actionnement d'un dispositif médical selon la revendication 15 faisant appel à l'appareil de commande (2) selon la revendication 2 ou l'une des revendications 2 et 3 à 11, dans lequel
l'appareil de commande (2) exécute l'action prédéfinie par rapport à la zone cible uniquement si le contrôle de plausibilité au point d'intersection est passé, ou exécute l'action prédéfinie sur la base du vecteur (10) déterminé par l'objet (8) et sa direction en cas de confirmation supplémentaire.

18. Procédé d'actionnement d'un dispositif médical selon la revendication 15 faisant appel à l'appareil de commande (2) selon la revendication 6 ou l'une des revendications 6 et 7 à 11, dans lequel le procédé comprend les étapes suivantes consistant à :
- détecter une direction de l'objet à partir de son orientation et de son extrémité,
- détecter l'élément du dispositif médical à activer, qui se trouve dans la direction opposée à celle de l'élément (8), et
- déclencher l'élément à activer, qui exécute l'action qui a été prédéfinie à partir de la direction de l'objet (8).
